(19) Europäisches Patentamt
European Patent Office
Office européen des brevets

(11) Publication number: **0 106 326 A1**

(12) **EUROPEAN PATENT APPLICATION**

(21) Application number: 83110236.3

(22) Date of filing: 13.10.83

(51) Int. Cl.³: C 07 C 79/35
C 07 C 93/14
//C07C125/065

(30) Priority: 15.10.82 JP 181665/82
15.10.82 JP 181666/82

(43) Date of publication of application: 25.04.84 Bulletin 84/17

(84) Designated Contracting States: CH DE FR GB IT LI

(71) Applicant: SUMITOMO CHEMICAL COMPANY, LIMITED
15 Kitahama 5-chome Higashi-ku
Osaka-shi Osaka-fu(JP)

(72) Inventor: Takahashi, Junya
4-2-303, Ryodo-cho Nishinomiya
Hyogo(JP)

(72) Inventor: Kato, Toshiro
D-410, 1-8, Sakasedai Takarazuka
Hyogo(JP)

(72) Inventor: Kamoshita, Katsuzo
2-3-11, Kofudai Toyono-cho
Toyono-gun Osaka(JP)

(74) Representative: Vossius Vossius Tauchner Heunemann Rauh
Siebertstrasse 4 P.O. Box 86 07 67
D-8000 München 86(DE)

(54) **1-Amino or nitro-3,5-disubstituted-4-ethoxybenzenes and their production.**

(57) A 1-amino or nitro-3,5-disubstituted-4-ethoxybenzenes are representable by the formula:

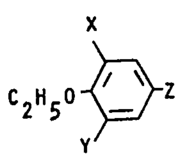

(I)

wherein X is a chlorine atom or a methyl group, Y is a methyl group, a methoxymethyl group or an ethoxy group and Z is an amino group or a nitro group, useful as intermediates in the production of isopropyl N-(3,5-disubstituted-4-ethoxyphenyl)carbamates of the formula:

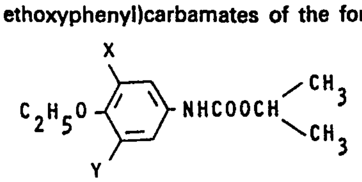

(II)

wherein X and Y are each as defined above, which are known to be per se useful as fungicides against phytopathogenic fungi, particularly those resistant to benzimidazole fungicides and thiophanate fungicides.

EP 0 106 326 A1

VOSSIUS · VOSSIUS · TAUCHNER
HEUNEMANN · RAUH
PATENTANWÄLTE
SIEBERTSTR. 4, 8000 MÜNCHEN 80

0106326

Sumitomo Chemical Company
Our Ref: S 626 EP

983

# 1-AMINO OR NITRO-3,5-DISUBSTITUTED-4-ETHOXYBENZENES AND THEIR PRODUCTION

The present invention relates to 1-amino or nitro-3,5-disubstituted-4-ethoxybenzenes and their production.

The said 1-amino or nitro-3,5-disubstituted-4-ethoxybenzenes are representable by the formula:

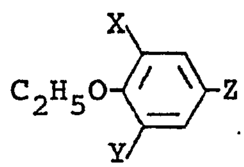

(I)

wherein X is a chlorine atom or a methyl group, Y is a methyl group, a methoxymethyl group or an ethoxy group and Z is an amino group or a nitro group.

The 1-amino or nitro-3,5-disubstituted-4-ethoxybenzenes (I) cover 3,5-disubstituted-4-ethoxyanilines (I: Z = $NH_2$) and 3,5-disubstituted-4-ethoxynitrobenzenes (I: Z = $NO_2$).

They are useful as intermediates in the production of isopropyl N-(3,5-disubstituted-4-ethoxyphenyl)carbamates of the formula:

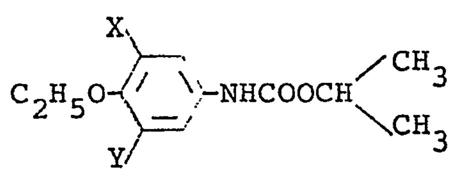

(II)

wherein X and Y are each as defined above, which are known to be per se useful as fungicides against phytopathogenic

fungi, particularly those resistant to benzimidazole fungicides and thiophanate fungicides. For instance, the reaction of 3,5-disubstituted-4-ethoxyanilines (I: Z = $NH_2$) with isopropyl chloroformate affords the corresponding isopropyl N-(3,5-disubstituted-4-ethoxyphenyl)carbamates (II).

The 3,5-disubstituted-4-ethoxynitrobenzene (I: Z = $NO_2$) can be produced by reacting the corresponding 3,5-disubstituted-4-nitrophenol of the formula:

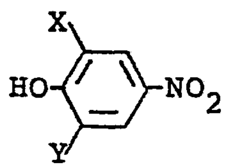

(III)

wherein X and Y are each as defined above, which is obtainable by the procedure as descirbed in J.Org.Chem., 27, 218 (1962), with an ethylating agent, usually in an inert solvent in the presence of a base at a temperature of 0 to 150°C for a period of not more than 10 hours.

Examples of the ethylating agent are ethyl bromide, ethyl iodide, diethyl sulfate, etc. Examples of the inert solvent are hydrocarbons (e.g. benzene, toluene, xylene), halogenated hydrocarbons (e.g. chloroform, carbon tetrachloride, dichloroethane), ethers (e.g. diisopropyl ether, dioxane, tetrahydrofuran), alcohols (e.g. methanol, ethanol, isopropanol), esters (e.g. ethyl acetate, butyl acetate), N,N-dimethylformamide, dimethylsulfoxide, sulforane, water, etc. Mixtures of two or more solvents as exemplified above are also usable. As the base, there may be used sodium hydride, sodium carbonate, potassium

carbonate, sodium hydroxide, potassium hydroxide, sodium ethoxide, sodium methoxide, etc.

Treatment of the reaction mixture by a per se conventional separation procedure such as distillation, recrystallization and chromatography gives the desired 3,5-disubstituted-4-ethoxynitrobenzene (I: Z = $NO_2$).

Reduction of the 3,5-disubstituted-4-ethoxynitrobenzene (I: Z = $NO_2$) by any procedure as conventionally adopted for conversion of a nitro group on the benzene ring into an amino group affords the corresponding 3,5-disubstituted-4-ethoxyaniline (I: Z = $NH_2$).

As the reduction procedure, there may be adopted, for instance, reduction with sodium sulfide or sodium hydrosulfide, reduction with a metal or catalytic reduction. When the reduction with sodium sulfide or sodium hydrosulfide is adopted, treatment of the nitrobenzene with the said reagent is usually carried out in a mixture of water and an alcohol (e.g. methanol, ethanol, isopropanol) at a temperature of room temperature to 100°C for a period of not more than 12 hours. The reduction with a metal is normally accomplished by treatment of the nitrobenzene with a metal (e.g. iron, tin, zinc) in the presence of an acid such as an inorganic acid (e.g. hydrochloric acid, sulfuric acid) or an organic acid (e.g. acetic acid) or its mixture with water at a temperature of room temperature to 100°C for a period of not more than 12 hours. In case of the catalytic reduction, the nitrobenzene is ordinarily treated with hydrogen gas in

the presence of a catalyst (e.g. Raney nickel, palladium, platinum dioxide, palladium-carbon, platinum black) in an inert solvent such as an alcohol (e.g. methanol, ethanol, isopropanol), an ether (e.g. diisopropyl ether, tetrahydrofuran, dioxane, ethylene glycol dimethyl ether) or an ester (e.g. ethyl acetate, butyl acetate) under a pressure of atmospheric pressure to 100 atm. at a temperature of room temperature to 100°C for a period of not more than 12 hours. The amount of the catalyst may be usually not less than 5 mol% on the basis of the nitrobenzene.

Recovery of the produced 3,5-disubstituted-4-ethoxyaniline (I: $Z = NH_2$) from the reaction mixture may be carried out by a per se conventional procedure such as chromatography, distillation or recrystallization.

Practical and presently preferred embodiments of the invention are illustratively shown in the following Examples.

Example 1

Sodium hydride (60 % dispersion in mineral oil) (18.38 g) was washed with hexane, and N,N-dimethylformamide (500 ml) was added thereto. To the resultant mixture kept at a temperature of -20 to -15°C, a solution of 2-chloro-6-methoxymethyl-4-nitrophenol (100.0 g) in N,N-dimethylformamide (200 ml) was dropwise added in 30 minutes. Temperature was elevated up to 28°C, ethyl iodide (214.8 g) was added thereto, and the resulting mixture was stirred at a temperature of 85 to 90°C for 15 minutes. The reaction

mixture was cooled to room temperature, ice water (1.5 L) was added thereto, and the resultant mixture was extracted with toluene (1 L). The toluene layer was washed with aqueous sodium hydroxide solution, diluted hydrochloric acid, aqueous potassium carbonate solution and water in order, dried over anhydrous magnesium sulfate and concentrated under reduced pressure. The residual oil was purified by silica gel chromatography using toluene to give 3-chloro-4-ethoxy-5-methoxymethylnitrobenzene (89.2 g). Yield, 79.1 %. $n_D^{25.0}$ 1.5412.

In the same manner as above, there were prepared a variety of 3,5-disubstituted-4-ethoxynitrobenzenes (I: Z = $NO_2$), of which typical examples are shown in Table 1.

Table 1

| Compound No. | X | Y | Physical constant |
|---|---|---|---|
| 1a | $CH_3$ | $CH_3$ | M.P. 57.5 - 58°C |
| 2a | $CH_3$ | $C_2H_5O$ | M.P. 66.5 - 67°C |
| 3a | Cl | $CH_3OCH_2$ | $n_D^{25.0}$ 1.5412 |
| 4a | Cl | $C_2H_5O$ | M.P. 69 - 70°C |
| 5a | $CH_3$ | $CH_3OCH_2$ | $n_D^{16.5}$ 1.5418 |
| 6a | Cl | $CH_3$ | NMR ($CDCl_3$) δ: 8.03 (d, 1H), 7.90 (d, 1H), 4.09 (q, 2H), 2.36 (s, 3H), 1.45 (t, 3H) |

Example 2

3,5-Dimethyl-4-ethoxynitrobenzene (1.95 g) was dissolved in ethyl acetate (50 ml), and 5 % palladium-carbon

(3.00 g) was added thereto. The resultant mixture was subjected to catalytic reduction with hydrogen under atmospheric pressure. After the absorption of hydrogen ceased, the reaction mixture was filtered to separate the catalyst. The filtrate was concentrated under reduced pressure, and the residual solid was washed with hexane to give 3,5-dimethyl-4-ethoxyaniline (1.58 g). Yield, 95.5 %. M.P., 74.5 to 75.5°C.

In the same manner as above, there were prepared a variety of 3,5-disubstituted-4-ethoxyanilines (I: $Z = NH_2$), of which typical examples are shown in Table 2.

Table 2

| Compound No. | X | Y | Physical constant |
|---|---|---|---|
| 1b | $CH_3$ | $CH_3$ | M.P. 74.5 - 75.5°C |
| 2b | $CH_3$ | $C_2H_5O$ | M.P. 59.5 - 60°C |
| 3b | Cl | $CH_3OCH_2$ | $n_D^{25.0}$ 1.5507 |
| 4b | Cl | $C_2H_5O$ | $n_D^{25.5}$ 1.5090 |
| 5b | $CH_3$ | $CH_3OCH_2$ | $n_D^{21.0}$ 1.5394 |
| 6b | Cl | $CH_3$ | NMR ($CDCl_3$) δ: 6.60 (d, 1H), 6.44 (d, 1H), 4.24 (b, 2H), 3.94 (q, 2H), 2.23 (s, 3H) 1.44 (t, 3H) |

What is claimed is:

1. A compound of the formula:

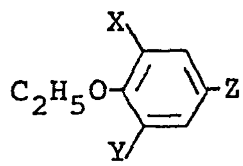

(I)

wherein X is a chlorine atom or a methyl group, Y is a methyl group, a methoxymethyl group or an ethoxy group and Z is an amino group or a nitro group.

2. The compound according to claim 1, wherein Z is an amino group.

3. The compound according to claim 1, wherein Z is a nitro group.

4. A process for producing a compound of the formula:

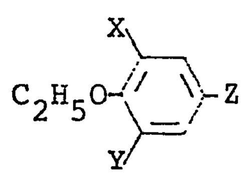

(I)

wherein X is a chlorine atom or a methyl group, Y is a methyl group, a methoxymethyl group or an ethoxy group and Z is an amino group or a nitro group, which comprises reacting a compound of the formula:

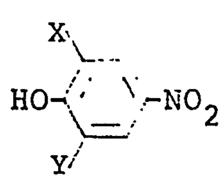

(III)

wherein X and Y are each as defined above with an ethylating agent to give the compound of the formula (I) wherein Z is a nitro group and X and Y are each as defined above and, when necessary, subjecting the latter to reduction by a per se conventional procedure for conversion of a nitro group on the benzene ring into an amino group to give the compound of the formula (I) wherein Z is an amino group.

5. A process for producing an aniline compound of the formula:

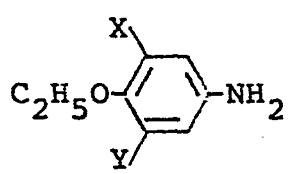

wherein X is a chlorine atom or a methyl group and Y is a methyl group, a methoxymethyl group or an ethoxy group which comprises subjecting a nitrobenzene compound of the formula:

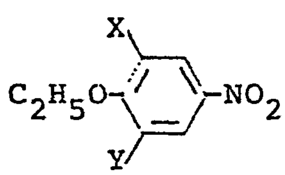

wherein X and Y are each as defined above to reduction by a per se conventional procedure for conversion of a nitro group on the benzene ring into an amino group.

6. The process according to claim 5, wherein the starting nitrobenzene compound is the one obtained by reacting a compound of the formula:

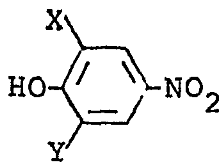

(III)

wherein X and Y are each as defined in claim 5 with an ethylating agent.

European Patent Office

# EUROPEAN SEARCH REPORT

0106326

Application number

EP 83 11 0236

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | GB-A- 899 930 (MONSANTO) * Claims; page 1, line 40 - page 2, line 22 * --- | 1 | C 07 C 79/35<br>C 07 C 93/14 //<br>C 07 C 125/065 |
| X | EP-A-0 063 905 (SUMITOMO) * Claims; pages 5,17,18; examples 51,57,110,115,139,208 * ----- | 1-6 | |
| | | | **TECHNICAL FIELDS SEARCHED (Int. Cl. 3)** |
| | | | C 07 C 79/00<br>C 07 C 93/00 |

The present search report has been drawn up for all claims

| Place of search | Date of completion of the search | Examiner |
|---|---|---|
| THE HAGUE | 20-01-1984 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82